# FASCICULE DE BREVET EUROPEEN

(11) **EP 0 487 674 B1**
(45) Date de publication et mention de la délivrance du brevet: **25.01.1995**
(21) Numéro de dépôt: 91910873.8
(22) Date de dépôt: 12.06.1991
(51) Int. Cl.: A61K 9/16, A61K 9/50, A61K 9/52

(54) **FORME GALENIQUE INJECTABLE RETARDEE**
INJIZIERBARE GALENISCHE RETARDZUBEREITUNG
INJECTABLE SLOW-RELEASE GALENICAL

(30) Priorité: 14.06.1990 FR 9007417
(43) Date de publication de la demande: 03.06.1992
(73) Titulaire: APLICACIONES FARMACEUTICAS, S.A. DE C.V., Mexico 03100 (MX)
(72) Inventeur: ANGELES URIBE, Juan, Valle de Aragon, Mexico 57100 (MX); JOSUE, Garza, Flores, Las Margaritas, Mexico 54050 (MX)
(74) Mandataire: Meylan, Robert Maurice
(86) Numéro de dépôt international: EP9101097
(87) Numéro de publication internationale: WO9119485

(56) Documents cités:
- WO-A-88/07816
- FR-A- 2 070 153

## Description

La présente invention concerne un procédé pour amélio rer le contrôle des propriétés pharmacocinétiques et pharmacologiques d'une substance pharmaceutiquement active injectable, ladite substance pharmaceutiquement active étant susceptible d'être administrée par injection parentérale à un mammifère, ou éventuellement d'autres animaux. Elle concerne également une microsphère solide, non poreuse, d'un diamètre compris entre 5 et 300 »m, comprenant au moins une telle substance pharmaceutiquement active contenue dans une structure sphérique formée par au moins une substance-support pharmacologiquement inactive, ainsi que l'utilisation d'une telle microsphère pour la fabrication d'une formulation destinée à l'administration parentérale par injection.

### Art antérieur

Des substances biologiquement actives, faiblement solubles en milieu physiologique, ont déjà été utilisées sous forme de suspension de particules et administrées par injection intramusculaire pour obtenir une dissolution lente, donc un effet prolongé dans l'organisme humain ou animal. Par exemple, des mélanges de norethistérone et de mestranol, sous forme de poudre cristalline, en suspension acqueuse, ont été testés en vue de la fabrication d'un anticonceptif injectable intramusculaire (J. Garza Flores et al, Contraception, mai 1988, Vol. 35, N° 5, 471 - 481).

Probablement à cause des variations de granulométrie et des irrégularités de forme des particules, ces compositions de l'art antérieur présentent en général plusieurs défauts :
- Courbe de libération des substances actives présentant un fort pic juste après l'injection, puis une pente descendante, ce qui augmente la dose totale nécessaire pour obtenir un effet durable suffisant.
- Formation occasionnelle de grumeaux ou croûtes dans la suspension.
- Nécessité d'utiliser des aiguilles hypodermiques de grand diamètre pour éviter le risque d'un blocage en sortie de seringue.

Dans certains cas, le contact direct de la particule active avec le tissu vivant environnant (très forte concentration locale) peut provoquer des réactions inflamatoires ou des lésions.

Des substances ayant une solubilité élevée en milieu physiologique ne peuvent pas être administrées sous cette forme si on désire obtenir un "effet retard". Une méthode connue pour ralentir la dissolution de telles substances est de les enrober ou de les micro-encapsuler.

Le brevet FR 2 070 153 (DUPONT DE NEMOURS) décrit des suspensions de particules de principes actifs enrobés de matrices de polymères polylactides. Elles sont obtenues soit par dépôt du polymère pré-dissout, soit par broyage du mélange pré-fondu. Ces techniques diminuent l'effet de choc médicamenteux initial et ralentissent la libération de la substance active. Cependant, les irrégularités de forme créent, là aussi, un risque d'incident opératoire au moment de l'injection, et les variations de forme, de taille, de composition et de structure interne de ces particules entraînent une variabilité non souhaitable des vitesses de dissolution dans l'organisme receveur, c'est-à-dire une dispersion des résultats ne permettant pas une prédiction pharmacocinétique précise.

Le brevet EP N° 257 368 (AMERICAN CYANAMID CO) décrit une composition à usage parentéral consituée de microsphères de graisses et/ou de cires, d'origines naturelle ou synthétique, à bas point de fusion (40° - environ 60°), chargées de particules d'un polypeptide, par exemple une hormone de croissance. Lorsque ces compositions sont injectées à des bovins, l'hormone de croissance voit sa dissolution retardée par l'enrobage de cire ou de graisse, ce qui entraîne une prolongation de sa présence dans l'organisme animal, entraînant une augmentation de la croissance ou de la lactation. Ces microsphères ont tendance à ramollir et à se déformer, s'agglutiner ou à coalescer lorsque la température ambiante est élevée, notamment dans les pays tropicaux (40-60°), ce qui peut entraîner des problèmes de manutention ou de stockage.

Comme la proportion de polypeptide actif, dans la particule, est limitée en pratique à 30-40 %, l'injection de ces particules présente également l'inconvénient d'injecter dans l'organisme une quantité de substance-support étrangère à cet organisme (cire d'abeille, graisse d'origine végétale, minérale ou synthétique, etc), au moins de l'ordre de 1,5 - 3 fois celle de la substance active.

D'autres techniques d'enrobage ou de micro-encapsulation ont été utilisées dans l'art antérieur dont certaines sont décrites, par exemple, dans "Encyclopédia of Chemical Technology, 3e édition, volume 15, pages 470 à 493 (1981), JOHN WILEY AND SONS. Les microcapsules ainsi formées contiennent souvent des particules "centrales" de taille très différente, voire pas de particule centrale du tout.

Le brevet EP N° 210 875 (TERAGENIX CORP.) décrit l'injection de microbilles de verre chargées radioactivement dans un corps, par exemple dans le foie, pour traiter localement une tumeur cancéreuse. La présence rémanente de telles billes, après traitement, ne se justifie que dans des cas graves.

L'élimination de la substance-support par les voies métaboliques du milieu intérieur peut être difficile; une telle substance est acceptable pour une administration orale (et une élimination par les voies digestives) en médecine humaine, mais beaucoup moins pour une injection parentérale en médecine humaine.

On a par ailleurs utilisé des hormones stéroïdes comme moyen de contraception, sous forme d'implants sous-cutanés. Ces implants d'une longueur de 0,5 à 2 cm peuvent être fabriqués par fusion et formage de l'hormone à l'état fondu, pure ou mélangée à un porteur lipoïdique. Ces procédés de fabrication sont décrits par exemple dans WO-88/078l6 (Endocon) ou dans le brevet US 4,244,949 (Gupta). La durée d'action de ces implants peut dépasser une année, par contre ce système est malcommode pour des durées d'action souhaitées de quelques semaines seulement. Il nécessite la mise en place par incision chirurgicale ou par trocart, et éventuellement l'enlèvement par excision.

### Sommaire de l'invention

Le but de la présente invention est de fournir des formulations à libération retardée pour l'administration par injection parentérale via une aiguille, à l'homme ou à d'autres mammifères, qui permettent une libération contrôlée des substances pharmacologiquement actives sans présenter les inconvénients des suspensions de particules ou des microcapsules de l'art antérieur.

Ce but est atteint grâce à un procédé consistant à mettre ladite substance sous forme de microsphères solides non poreuses d'un diamètre compris entre 5 et 300 microns comprenant au moins une substance pharmaceutiquement active contenue dans une structure sphérique formée par au moins une substance-support pharmacologiquement inactive, ladite substance-support formant la structure sphérique étant naturellement présente dans l'organisme dudit mammifère, étant stable à l'état solide jusqu'à une température d'au moins 60°C et dans le milieu physiologique dudit mammifère, la cinétique de dissolution de la substance-support dans l'organisme du mammifère receveur étant plus lente que la cinétique de libération de la substance active dans ce même organisme, et à séparer lesdites microsphères en fractions calibrées selon leurs diamètres, et grâce à l'utilisation de ces fractions pour la fabrication d'une formulation destinée à l'administration parentérale par injection.

Par stable à l'état solide, jusqu'à 60°C, il faut entendre que non seulement les microsphères ne fondent pas, mais aussi qu'elles ne ramollissent et ne s'agglutinent pas.

Par stable à l'état solide, au sens de la présente invention, il ne faut également pas entendre la stabilité indéfinie que présenterait par exemple une microsphère de verre, mais, suffisamment stable pour que l'ordre de grandeur de la durée de vie dans ce milieu (la disparition des sphères s'opérant par simple dissolution lente ou par attaque chimique métabolique) ne soit pas inférieure à la durée d'action désirée du médicament: en d'autres termes, la cinétique de dissolution de la substance-support dans l'organisme de mammifère receveur doit être plus lente que la cinétique de libération de la substance active dans ce même organisme.

Si on utilise un procédé de pulvérisation-congélation pour la fabrication des microsphères, la substance-support doit être chimiquement stable à sa température de fusion, ainsi qu'à l'état fondu, dans au moins un domaine de température suffisant pour cette opération.

La vitesse de dissolution d'une microsphère dans un milieu-solvant donné est fonction du rayon de la sphère (compte tenu des relations liant volume, surface et rayon d'une sphère). Selon un aspect de la présente invention, le fait d'utiliser des sphères solides non poreuses permet d'avoir une connaissance précise de la relation masse-surface des particules et donc, grâce à une sélection du calibre des sphères, c'est-à-dire du rayon ou d'une répartition de rayons, de maitriser un paramètre de contrôle du taux de libération du ou des principes actifs administrés.

La vitesse de dissolution des principes actifs dépend également de la structure de la microsphère, et en particulier de l'accessibilité de son intérieur au solvant. Une substance inactive prenant par exemple la structure d'un gel gonflé en milieu physiologique (par exemple une fraction de collagène) ne provoque qu'un faible retard dans la libération des substances actives, alors qu'une substance ayant une structure hydrophobique compacte fournit un effet retard maximal. Selon un autre aspect de la présente invention, la sélection de la molécule déterminant et structurant la microsphère permet d'obtenir une libération des principes actifs retardée, variant de quelques heures à plusieurs semaines.

Si la substance pharmacologiquement active se trouve sous forme de solution solide dans la substance-support ou de suspension pratiquement homogène de particules dispersées, petites (par ex. colloïdes) par rapport au diamètre de la microsphère (10 - 300 »m), sa dissolution est ralentie, en fonction des concentrations respectives. Si la substance active se trouve sous forme de particules plus grosses, enrobées dans une couche extérieure d'une substance hydrophobe le début de la dissolution est retardé. Pour des raisons techniques de fabrication, la taille des particules enrobées est limitée par rapport au diamètre des microsphères : dans le procédé de pulvérisation/congélation, il est préférable de limiter la taille des particules à 1/10 de celui des microsphères. L'ajustement de ces différents paramètres permet, selon l'invention, un contrôle précis de la libération des principes actifs.

Cette même précision de controle, en évitant les surdosifications ou le besoin de compenser des sous-dosifications, permet de réduire la quantité totale prévue pour l'administration de la ou des substances biologiquement actives à la quantité minimum requise pour obtenir l'effet thérapeutique désiré et de diminuer ainsi le risque de produire chez le malade des effets secondaires indésirables.

Une dose injectable de 200 mg de microsphères, par ampoule, contenant jusqu'à 50 mg de principe actif, est à considérer comme raisonnable.

Certaines substances additives de l'association peuvent ne pas être directement actives sur l'organisme receveur, du moins dans l'application visée, ni constituer la base de la structure sphérique. L'association peut comprendre divers moyens pharmaceutiquement acceptables pour améliorer la stabilité ou l'intégrité chimique des substances ou de l'ensemble de la structure : surfactants, antioxydants, antimicrobien, tampon, etc. En particulier, il peut s'avérer utile d'abaisser un point de fusion ou d'inhiber une réaction de décomposition durant le procédé de fabrication, (par ex. fusioncongélation) des microsphères.

Par rapport aux suspensions de principes actifs purs sous forme de particules de formes irrégulières connues dans l'art antérieur, les microsphères selon la présente invention ont l'avantage d'avoir moins tendance à s'agglutiner et de passer de manière plus fluide par une aiguille hypodermique. D'autre part, des microsphères peuvent être triées et séparées de manière plus fine et plus fiable en fonction de leur taille, que des particules de formes irrégulières.

La forme galénique selon la présente invention peut se présenter sous forme de poudre de microsphères en flacons-ampoules, prête à être mise en suspensions, ou sous forme de suspensions déjà préparées, conditionnées en ampoules injectables, ou directement en seringues, prête à être administrée en médecine humaine ou vétérinaire. Le milieu de suspension peut être de l'eau, une solution saline, une huile contenant les tampons, surfactants, conservants, habituellement utilisés dans les suspensions injectables par les pharmaco-techniciens, ou toute autre substance ou combinaison qui ne menace pas l'intégrité physique et chimique des substances en suspension et qui convienne pour l'organisme qui la recevra. Si l'on souhaite éviter une élévation initiale brusque du taux de principe actif dans le milieu intérieur de l'organisme receveur, on préfèrera, dans le cas des suspensions prêtes à l'emploi, l'utilisation de vecteurs liquides dans lequel lesdits principes actifs sont pratiquement insolubles. Dans le cas de substances actives partiellement solubles dans le vecteur liquide tiède, mais insolubles à froid, on préfèrera, sur le plan pharmacologique, éviter la formation de précipités (dit effet de "caking") en réalisant des formulation présentant séparément les microsphères en poudre et le vecteur liquide qui ne seront mélangés qu'au moment de l'injection.

Dans les applications vétérinaires, où la durée d'effet désirée peut être très longue (par exemple période de lactation de la femelle adulte), on peut utiliser des diamètres de quelques centaines de microns. Si on souhaite limiter le diamètre des aiguilles de seringues d'injection pour le confort du patient, il est bon de limiter le diamètre des microsphères à 300 micrometers (microns) et plus préférablement à 100 microns. Par contre, pour des durées d'effet très courtes (par exemple circadiennes), le diamètre d'une microsphère peut s'abaisser à 5 microns.

Pour la plupart des applications en médecine humaine (durée d'action du principe actif comprise entre un cycle circadien et un cycle mensuel), il est préférable d'utiliser des microsphères dont le diamètre soit compris entre 10 et 100 micrometers (microns), selon les combinaisons de substances actives/substances-support.

Un tri des microsphères selon leur diamètre peut être réalisé lors de la fabrication à l'aide de procédés connus : par exemple, par séparateurs cycloniques, par tamisage avec succion d'air ou encore par tamisage en milieu acqueux. En pratique, il suffit que plus de 70 % des microsphères aient des diamètres compris entre 70 % et 130 % d'un diamètre spécifié. Si besoin, la courbe de dissolution idéale, déterminée par l'application envisagée, peut être approchée en effectuant un mélange de lots ayant des diamètres différents appropriés. De plus, les particules non conformes aux spécifications peuvent être recyclées.

Des procédés pour mettre un produit solide sous forme de microsphères, par abrasion mécanique, sont connus dans l'état de la technique. D'autres procédés utilisent, par exemple, la mise en suspension du produit à l'état fondu sous forme de microgouttes, sous agitation, dans un vecteur liquide avec lequel ledit produit est non miscible, suivi de la solidification dudit produit. Pour réaliser les microsphères selon la présente invention, on a préféré développer un procédé qui consiste à pulvériser sous pression et/ou à l'aide de gaz chaud, (éventuellement avec vibrations) la substance support destinée à constituer les sphères, à l'état fondu, dans laquelle les substances pharmacologiquement actives ont été dispersées et se trouvent soit à l'état dissous, soit sous forme de particules < 5 »m, puis à congeler rapidement le brouillard ainsi formé. Il est cependant difficile de fabriquer des microsphères, contenant des particules solides enrobées, de diamètre < 2 »m par un procédé de pulvérisation-congélation.

Des substances support, parmacodynamiquent inactives selon l'invention, dont la températures de fusion est supérieure à environ 70° et qui sont ou peuvent être rendues thermostables au-dessus de leur point de fusion pour pouvoir subir le procédé de fabrication, permettent de réaliser des microsphères stables (pas de ramollissement, d'agglutination) à des températures ambiantes basses ou moyennes.

Cependant, dans la mesure où le principe actif en suspension supporte des températures élevées sans décomposition il est préférable, pour éviter un risque d'altération des microsphères lors d'une élévation accidentelle importante de température (transport, stockage), de choisir une substance-support dont la température de fusion est supérieure à 90°C, pour constituer la structure de la microsphère.

Parmi les substances support préférées, on citera comme substances inactives pouvant constituer la structure de microsphères:
1. Coprostérol, dont le point de fusion est 101° C, produit obtenu par métabolisation des stérols et qui participe à la formations des stéroïdes et des acides biliaires.
2. Acide Glycocholique, dont le point de fusion est de 130° C, qui fait partie des sels biliaires.
3. Cholestérol, dont le point de fusion est de 148 - 149° C, principal stérol chez les mammifères, présent dans presque tous les tissus de l'organisme humain, et ses esters.

Il est à première vue surprenant d'injecter du cholestérol en suspension de particules sphériques à un être humain, vue le rôle qu'on lui attribue dans certaines maladies cardiovasculaires. Mais par rapport aux 5-10 gr. naturellement présents à l'état libre dans le milieu physiologique, les 50-200 mg. d'une injection sont une quantité faible. De plus, sa relative inertie métabolique permet de le considérer comme pharmacodynamiquement inactif à ces doses. D'autre part, ses propriétés physiques en fait une excellente substance-support dans le cadre de la présente invention.

Les substances pharmacologiquement actives qui conviennent particulièrement pour ce système galénique sont celles qui 1/ sont solubles (ou réactives) dans les fluides biologiques, 2/ qui, même si elles tendent à se décomposer aux températures très élevées, proches ou supérieures à leur point de fusion, restent physiquement et chimiquement stables au point de fusion de la substance inactive qui sert de structure:
Un exemple est le méthoclopramide, un antiémétique librement soluble dans l'eau.

Un autre exemple est la morphine base, analgésique narcotique, dont le point de fusion est 254 - 256°C, et qui devient physiquement et chimiquement instable vers 200° C. La morphine (particules de l'ordre de 1 »m à 5 »m, ou plus petites) est dispersée dans du cholestérol liquide (TF = 148°C) sans risque de décomposition. Le mélange est pulvérisé, congelé en microsphères. Sous cette forme, la morphine peut, par exemple, être administrée à raison d'une injection quotidienne, en milieu hospitalier, de 50 mg morphine contenus dans 200 mg microsphères/ampoule (dose fortement variable à cause des réactions individuelles à la morphine) ; pour un patient ambulatoire, on préfèrera adapter la présentation (dose et structure des sphères) pour une injection tous les 2-3 jours, voire hebdomadaire.

Parmi les substances actives trop solubles en milieu physiologique pour pouvoir être administrées à l'état libre en présentant un effet retard, et suffisamment stables à chaud pour être incorporées dans du cholestérol, on citera notamment:
a) substances agissant sur le système nerveux central (tranquillisants, comme le LORAZEPAM, l'HALOPERIDOL, antiparkinsoniens, comme le BIPERIDEN, le TRIHEXYPHENIDYL HCL, anticonvulsifs, comme le CLONAZEPAM, narcotiques, comme la MORPHINE BASE)
b) sur le système neurovégétatif (antiémétiques, comme la METHOCLOPRAMIDE le MALEATE d'ACEPROMAZINE, gastrocinétiques, comme la DOMPERIDONA)
c) vasodilatateurs, périfériques comme la VINCAMINE, la NYLIDRINE HCL, la FLUNARIZINE, le PIZOTIFENE, la DIHYDROERGOTAMINE, ou bronchiques comme le BROMOHYDRATE le FENOTEROL, le TOLBUTEROL, le CLENBUTEROL, le SALBUTAMOL
d) antihistaminiques (ASTEMIZOLE, MALEATE de CHLORFENAMINE, AZATADINE)
e) antagoniste des récepteurs H2, la FAMOTIDINE
f) plusieurs stéroïdes (DEXAMETHASONE, BETAMETHASONE) conviennent également.

La dissolution de certains analgésiques, en soi peu solubles, comme l'indométacine ou la naproxène, peut être ralentie d'avantage par incorporation dans une structure de cholestérol (ce qui permet d'espacer les injections, en augmentant les doses unitaires).

La présente invention sera mieux comprise à l'aide des figures et des exemples qu'elles illustrent.

### Brèves description des figures

La figure 1 montre le schéma de fabrication de microsphères de cholestérol selon la présente invention.
La figure 2 montre une microphotographie (microscope électronique) de microsphères de cholestérol.
La figure 3 montre la répartition granulométrique d'une fraction de microsphères de cholestérol (diamètre moyen 15 »m)
La figure 4 montre la répartition granulométrique d'une fraction de microsphères de cholestérol (diamètre moyen 25 »m)
La figure 5 représente un montage expérimental pour déterminer la vitesse de dissolution de microsphères.
Les figures 6 et 7 montrent le profil de dissolution de microsphères (fig. 6) de 17-β-estradiol supporté par du cholestérol, comparé au profil de dissolution de cristaux de 17-β-estradiol (fig. 7).
Les figures 8 et 9 montrent le profil de dissolution de microsphères de diazepam supporté par du cholesterol, comparé au profil de dissolution de cristaux de diazepam.
La figure 10 montre les niveaux plasmatiques de diazepam obtenus (chez le lapin) par injection respectivement d'une solution (courbe 0) de cristaux en suspensions (courbe 1) et de microsphères de diazepam/cholestérol (courbe 2).
Les figures 11, 12, 13, montrent les niveaux plasmatiques de 17-β-estradiol obtenus (chez le lapin) par injection respectivement d'une solution (courbe 0) de cristaux en suspensions (courbe 1) et de microsphères de 17-β-estradiol/cholestérol (courbe 2).

### Exemple 1 : fabrication de microsphères de cholestérol.

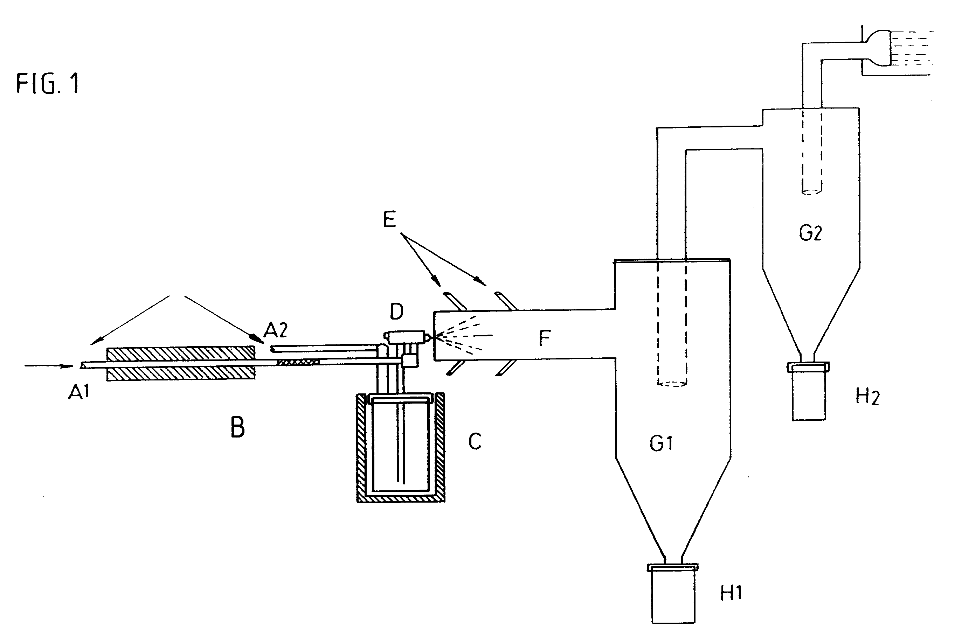

Nous nous référons à la figure 1. De l'azote préchauffé sous pression est envoyé par le tube d'entrée A₁ dans le dispositif pulvérisateur et traverse une zone de chauffage B thermo-régulée où il est porté à une température comprise entre 160 et 190°C, avant d'être admis dans le pulvérisateur D. Le pulvérisateur D est relié par une tubulure à une enceinte C chauffée dans laquelle le cholestérol est maintenu à l'état fondu (T = 150°C) et sous pression d'azote (entrée A₂). Il est entraîné par le courant d'azote et mélangé à celui-ci, pour être pulvérisé en brouillard par la buse de sortie du pulvérisateur D et pénètre dans la chambre de pulvérisation-congélation F. Un réservoir E contient de l'azote liquide qui s'évapore et pénètre par plusieurs canalisations sous forme de gaz ultra froid, à grande vitesse, dans la chambre de pulvérisation-congélation F, où il rencontre le brouillard de cholestérol. Les gouttelettes, sitôt après leur formation par le pulvérisateur sont entourées d'un courant de gaz glacial qui les cristallise en microsphères et les empêche de toucher les parois avant leur solidification totale. La température à la sortie de la chambre de pulvérisation-congélation est comprise entre - 15°C et - 50°C. La totalité des microsphères produite à l'aide de cette chambre F a une forme sphérique parfaite. A la sortie de cette chambre F se trouvent deux séparateurs cycloniques, G₁ , G₂, connus par ailleurs montés en séries.

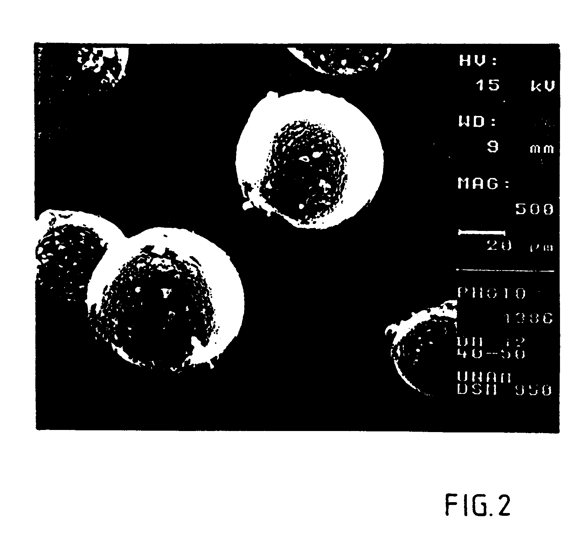

Les microsphères sont récupérées dans des récipients collecteurs H₁, H₂ ; les gaz, à la sortie des cyclones, traversent un filtre décontaminant I, dans lequel une légère dépression par rapport à la pression régnant dans le premier cyclone est maintenue à l'aide d'une pompe. La figure 2 montre une fraction de microsphères de cholestérol récupérées, en microphotographie (au microscoque électronique).

### Exemple 2 : Répartition granulométrique

Les microsphères de cholestérol, fabriquées dans les conditions opératoires ci-dessus, sont séparées en fractions.

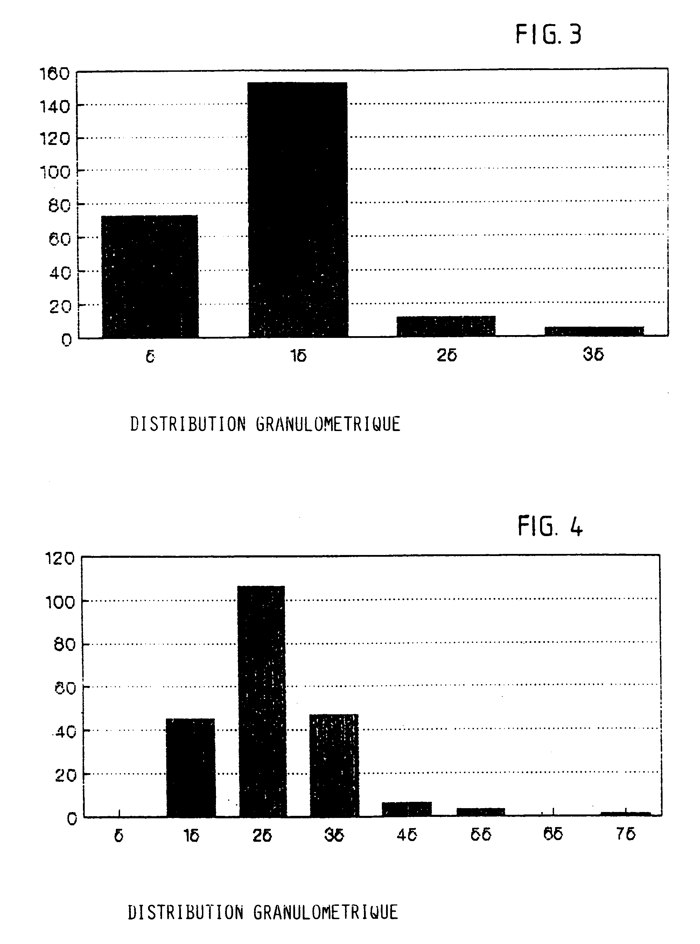

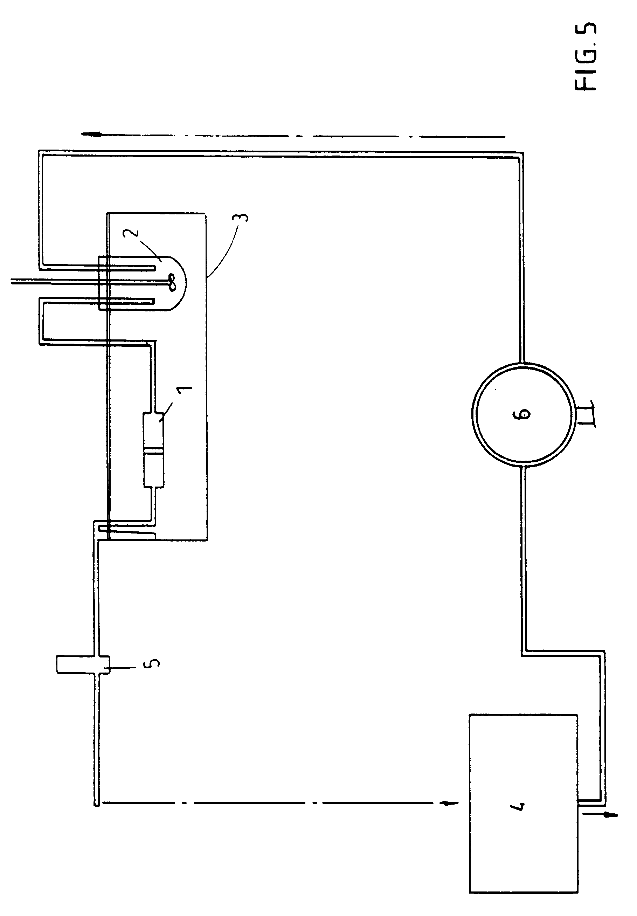

Les figures 3 et 4 montrent les distributions granulométriques de fractions centrées respectivement sur 15 »m et 25 »m.

### Exemple 3 : Fabrication de microsphères de 17-β-estradiol/cholestérol.

On applique le procédé de l'exemple l à un mélange 17-β-estradiol/cholestérol dans un rapport pondéral 1/9
Conditions opératoires :
Fusion : 149°C en atmosphère d'azote.
Aspersion : par valve, avec pression d'air de 2,5 psi (200 gr/cm²)
Congélation : par air à -20°C, sous pression de 4 kg/cm²
Recouvrement : par cyclones
Sélection : en milieu acqueux et par criblage selon la granulométrie.

### Exemple 4 Fabrication de microsphères de diazepam/cholestérol.

On applique le procédé de l'exemple 1 à un mélange diazepam//cholestérol dans un rapport pondéral 1/2.
Conditions opératoires :
Fusion : 138°C en atmosphère d'azote.
Aspersion : par valve, avec pression d'air de 1,5 psi (100 gr/cm²)
Congélation : par air à -20°C, sous pression de 4 kg/cm²
Recouvrement : par cyclones
Sélection : en milieu acqueux et par criblage selon la granulométrie.

### Exemple 5 : Fabrication de microsphères de caféine/cholestérol.

On applique le procédé de l'exemple 1 à un mélange caféine/cholestérol dans un rapport pondéral 20:80.
Conditions opératoires :
Fusion : 165°C en atmosphère d'azote.
Aspersion : par valve, avec pression d'air de 2,0 psi (140 gr/cm²)
Congélation : par air à -20°C, sous pression de 4 kg/cm²
Recouvrement : par cyclones
Sélection : en milieu acqueux et par criblage selon la granulométrie.

Analyses comparatives spectrophotométrique UV et IR avant et après formation de microsphères.

Il est nécessaire de vérifier qu'aucune dégradation chimique des substances n'intervient au cours du processus de fusion-congélation, qui puisse modifier leur action thérapeutique. La comparaison se fait entre la matière première (cristaux) et les microsphères obtenues par fusion-congélation, par analyse spectrophotométrique en UV et en IR. Les graphiques "avant et après" doivent toujours être superposables en UV et généralement IR. Lorsqu'il apparait des différences dans les courbes d'infrarouge, il convient de vérifier si elles n'ont pas été causées par un phénomène de polymorphisme, en ayant recours à la chromatographie liquide de haute résolution avec arrangement de diodes. Il convient également de recourir à la thermographie, non seulement pour bien cerner les points de fusion, mais aussi pour déterminer s'il y apparaissent des endothermies ou exothermies qui peuvent aussi bien exprimer des modifications structurelles ou un polymorphisme, susceptibles d'avoir un effet sur le processus de formation des microsphères, que les dégradations chimiques produites par le chauffage.

Appareil utilisé en spectrographie en ultraviolet : Hewlett Packard modèle 8452A avec arrangement de photodiodes, avec cellule de quartz ayant un faisceau de 0,1 cm.

Solvents : ethanol pour le 17-β-estradiol et le cholestérol; HC1 à 0,1 N pour le diazepam.

Les résultats ne montrent pas de trace d'alération.

Appareil utilisé en spectrophotométrie en infrarouge : Nicolet 205 FT-IR. Milieu de dispersion : bromure de potasse
Chromatographe liquide de haute résolution avec arrangement de diodes : marque : WATERS, avec Photodiode (array detector) mod. Waters 990 et N.E.C. Powermate 2 workstation.

Thermographe : SHIMADZU DSC-50 Calormeter et CR4A workstation.

Les résultats ne montrent aucune altération après la mise en microsphères pour le 17-β-estradiol et le diazepam.

Le montage expérimental pour effectuer les tests de dissolution in vitro est montré par la figure 5. Une cellule à perfusion 1, contenant l'échantillon, est alimentée par un réservoir (agité) de milieu de dissolution 2; les deux sont contenus dans un bain-marie 3. La densité optique du milieu est enregistrée par un spectrophotomètre 4 et le milieu est ramené dans le réservoir. Un piège à bulle 5 et une pompe péristaltique 6 complètent le circuit. Les exemples suivants montrent la reproductibilité comparée des parties initiales des courbes de dissolution de cristaux et de microsphères de granulométrie comparable, d'un même produit. L'appareil utilisé est celui de la figure 5. Plusieurs (de 3 à 6) circuits de mesure (cellules de dissolution et tubulures) contenant des échantillons identiques, sont mis en oeuvre en parallèles par la même pompe péristaltique et mesurés simultanément.

### Exemple 5 : Dissolution de microsphères 17-β-estradiol/cholestérol (1/9):

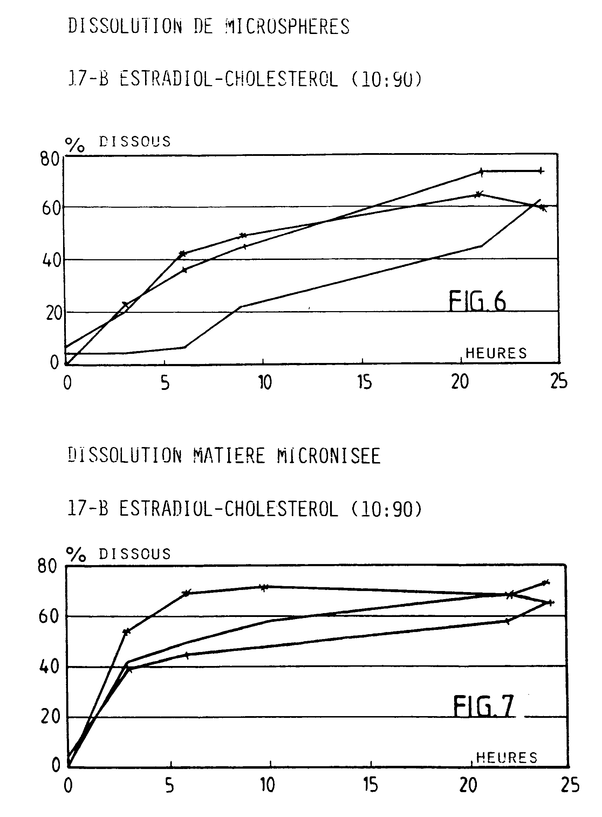

L'appareil utilisé est celui de la figure No 5
Milieu de dissolution utilisé: H₂O qualité HPLC avec 0.01% de Tween 80
Echantillon: 50mg
Granulométrie: 50 à 100 microns
Intervalles d'échantillonage: 0,3,6,9,12,24 heures Longueur d'ondes de spectrophotomètrie: 282 nm
Les courbes de dissolution sont montrées dans les figures 6 (microsphères) et 7 (cristaux).

### Exemple 6 : Dissolution de microsphères diazepam/cholestérol (1/2):

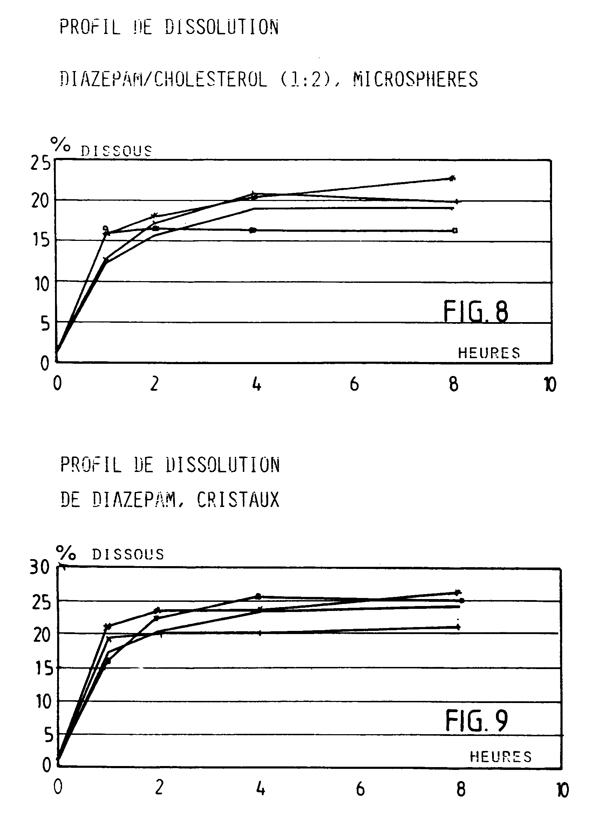

L'appareil utilisé est celui de la figure No 5
Milieu de dissolution utilisé: H₂O qualité HPLC avec 0.01% de Tween 80
Echantillon: 50mg
Granulométrie: 50 à 100 microns
Intervalles d'échantillonage: 0,1,2,4,8 heures
Longueur d'ondes de spectrophotomètrie: 286 nm
Les courbes de dissolution sont montrées dans les figures 8 (microsphères) et 9 (cristaux).

### Exemple 7 : Formulations

| Formule No 1 | |
|---|---|
| Microsphères de 17-β-estradiol/cholestérol* | 25 mg |
| Polyethylène Glycol 800 | 20 mg |
| Carbdoxymethylcélulose sodique | 1.66 mg |
| Polysorbate 80 | 2.0 mg |
| Propylparabène | 0.14 mg |
| NaCl | 1.2 mg |
| H₂O cbp | 1 ml |

| | |
|---|---|
| *equivalents à 2.5 mg de 17-β-estradiol | |

| Formule No 2 | |
|---|---|
| Microsphères de diazepam/cholestérol 33% | 33.3 mg |
| Polyethylène Glycol 4000 | 3.0 mg |
| NaCl | 8.5 mg |
| Alcool de benzyl | 9.0 mg |
| Hydroxide de Soude ou HCl: | suff. pour pH 5-6 |
| H₂O cbp | 1 ml |

| | |
|---|---|
| *Equivalents à 10mg de diazepam. | |

### Exemple 8 : Etude des niveaux plasmatiques de 17-β-estradiol/cholestérol chez le lapin

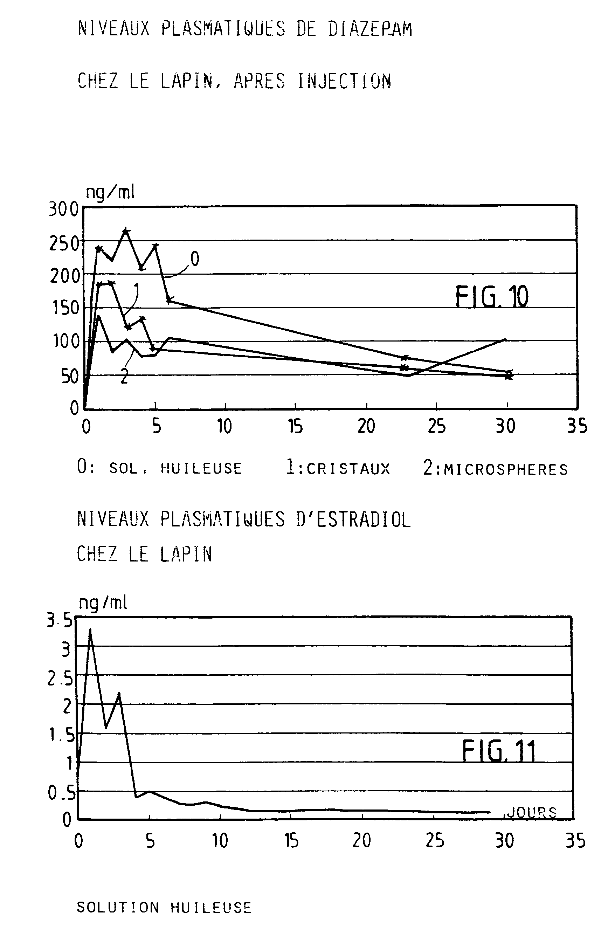

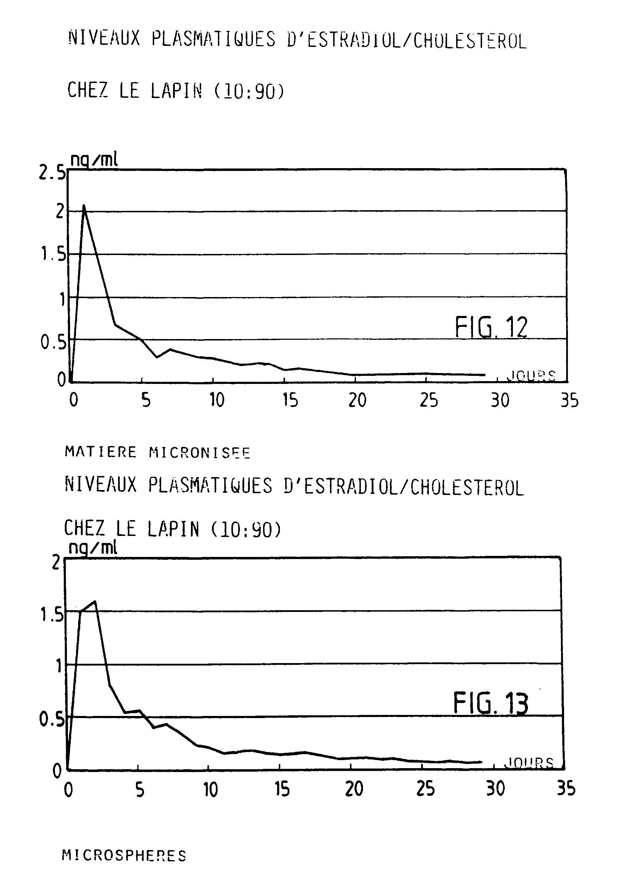

L'étude comprend l'évaluation comparée de l'effet sur les niveaux plasmatiques chez le lapin, produit par l'administration parentérale d'estradiol sous forme d'une solution huileuse (fig. 11), d'une suspension acqueuse de particules d'estradiol (fig. 12), et d'une suspension aqueuse de microsphères (fig. 13) d'estradiol/cholestérol (formule No 1).

A 10 lapins mâles de race Nouvelle Zélande d'un poids moyen de 3.5 Kg on administre une dose unique intramusculaire de 5mg d'estradiol.

L'intervalle d'échantillonage est de 1,2,4 et 24 heures durant 20 jours, puis à chaque trois jours jusqu'à atteindre 30 jours.

Les prises sont de 2ml par venoponction, sont centrifugées puis gardées à 20 degrés C jusqu'à son analyse par radioimmunoanalyse.

### Exemple 9 : Evolution comparative des niveaux plasmatiques de diazepam en solution huileuse, en suspension de cristaux et en suspension de microsphères.

Sujets d'expérimentation: lapins de race Nouvelle Zélande agés d'environ 5 mois et pesant en moyenne 3.7 Kgs.

La prise de référence est de 5 ml de sang par ponction cardiaque, suivie de l'administration intramusculaire de 2 ml de la formule à tester dans le membre inférieur droit (formule N^{°} 2). Les "cristaux" servant à la comparaison sont constitués d'un mélange 1:2 fondu, recongelé et broyé de diazepam/cholestérol.

Les prises à analyser furent prélevées à intervalles de 30 min. durant 2 heures et à intervalles de 60 min. jusqu'à compléter 6 heures. Dans certains cas, en fonction des caractéristiques cinétiques du médicament, il y eut des prises additionnelles.

Des prises à analyser de 2 ml, également prélevées par ponction cardiaque, furent placées en Vacutainer, additionnées d'héparine, centrifugées à 3000 rpm durant 10 min., puis le plasma séparé et congelé en cryotubes à 20 degrés C jusqu'à son analyse.

### Conditions Chromatographiques:

Détection UV à 220 nm
Colonne Novapack C₁₈ de 10 microns
Phase mobile: buffer phophates pH 3.5/acetonitryle
59:41 V/V
Flux: 1.6 ml/min
Standard interne: ibuprofen
La figure 10 montre l'évolution des niveaux plasmatiques jusqu'à 30 heures après l'injection obtenus respectivement par injection d'une solution huileuse (courbe 0), d'une suspension de microsphères selon la formule N^{°} 2 de l'exemple 8 (courbe 2) et d'une suspension équivalente de cristaux (courbe 1). on constate que l'utilisation de microsphères élimine le pic plasmatique initial.

En résumé, l'ensemble des résultats ci-dessus montre que dans la phase initiale de dissolution, des substances pharmaceutiquement actives présentent des valeurs numériques plus reproductibles et un profil de dissolution plus régulier lorsqu'elles sont sous forme d'échantillon de micro-sphères calibrées que sous forme de particules de formes irrégulières. Ceci permet de calculer de manière plus précise une dose pharmaceutiquement efficace. De plus, la disparition, ou du moins la forte diminution du pic initial de dissolution (par rapport à des cristaux ou des particules quelconques) ainsi que le ralentissement et la prolongation globale du phénomène de dissolution permet de calculer des doses unitaires plus importantes destinées à être administrées à des intervalles de temps plus espacés.

D'autre part, les résultats ci-dessus montrent que l'utilisation de ce type de structure convient aussi bien à la fabrication des médicaments dont la durée d'action est relativement courte, quelques heures à quelques jours (p.e. analgésiques) qu'à des substances dont la durée d'action envisagée est de plusieurs semaines. Parmi ces dernières on peut citer en particulier l'utilisation d'hormones sexuelles (comme la progestérone et le 17-β-estradiol) pour la fabrication d'anticonceptifs destinés à une injection mensuelle ou d'anticonceptifs destinés plus particulièrement à la femme post-partum, ou encore pour la fabrication de médicaments à longue durée d'action, injectables, destinés à la prévention de l'ostéoporose chez la femme ménopausée.

Le procédé de fabrication décrit ci-dessus, les structures sphériques et les formulations obtenues et leur utilisation par voie parentérale par injection ne sont bien entendu pas limitées aux substances données en exemples ci-dessus, mais sont applicables à toutes substances pharmacologiquement actives, chimiquement stables pendant la micronisation, à condition que les modifications pharmacocinétiques que permettent les microsphères (durée brève ou longue selon le diamètre, régularisation des profils plasmatique), présentent un avantage thérapeutique ou de commodité et que les doses à administrer ne dépassent pas un volume raisonnable. On peut choisir le mode d'administration parmi l'injection hypodermique, l'injection sous cutanée, l'injection intramusculaire, l'injection intra-articulaire et l'injection intra-rachidienne, selon l'application envisagée.

## Revendications

1. Microsphère solide, non poreuse, d'un diamètre compris entre 5 et 300 »m, comprenant au moins une substance pharmaceutiquement active contenue dans une structure sphérique formée par au moins une substance-support pharmacologiquement inactive, ladite substance pharmaceutiquement active étant susceptible d'être administrée par injection parentérale à un mammifère, caractérisée en ce que ladite substance-support formant la structure sphérique est naturellement présente dans l'organisme receveur, est stable à l'état solide jusqu'à une température d'au moins 60°C et dans le milieu physiologique receveur, et en ce que la cinétique de dissolution de la substance-support dans l'organisme du mammifère receveur est plus lente que la cinétique de libération de la substance active dans ce même organisme.

2. Microsphère selon la revendication 1 caractérisée en ce que le diamètre de ladite microsphère est compris entre 10 et 100 »m.

3. Microsphère selon la revendication 2 caractérisée en ce que sa composition comprend également des additifs pharmaceutiquement acceptables.

4. Microsphère selon l'une des revendications 1 à 3, caractérisée en ce que ladite substance-support est choisi parmi les substances chimiquement stables à l'état fondu, que la substance pharmaceutiquement active est choisie parmi les substances chimiquement stables dans la substance-support fondue, et en ce que ladite microsphère est obtenue par la dispersion de ladite substance pharmaceutiquement active dans ladite substance-support à l'état fondu, suivi de la pulvérisation du mélange obtenu et la congélation rapide des gouttelettes dans un gaz froid.

5. Microsphère selon la revendication 4 caractérisée en ce que la substance-support est choisie parmi le coprostérol, l'acide glycocolique, le cholestérol et les esters de cholestérol.

6. Microsphère selon la revendication 5, caractérisée en ce que la substance pharmaceutiquement active est choisie parmi les substances agissant sur le système nerveux central comme tranquillisant, notamment le LORAZEPAM, l'HALOPERIDOL et le DIAZEPAM, comme antiparkinsoniens, notamment le BIPERIDEN, le TRIHEXYPHENIDYL HCL, comme anticonvulsifs, notamment le CLONAZEPAM, comme narcotiques, notamment la MORPHINE BASE et les dérivés de morphine.

7. Microsphère selon la revendication 5, caractérisée en ce que la substance pharmaceutiquement active est choisie parmi les substances agissant sur le système neuro-végétatif, comme antiémétiques, notamment la METHOCLOPRAMIDE, le MALEATE d'ACEPROMAZINE, comme gastrocinétiques, notamment la DOMPERIDONA.

8. Microsphère selon la revendication 5 caractérisée en ce que la substance pharmaceutiquement active est choisie parmi les vasodilatateurs périphériques, notamment la VINCAMINE, la NYLIDRINE, la FLUNARIZINE, le PIZOTIFENE, la DIHYDROERGOTAMINE, et bronchiques, notamment le BROMOHYDRATE, le FENOTEROL, le TOLBUTEROL, le CLENBUTEROL, le SOLBUTAMOL.

9. Microsphère selon la revendication 5 caractérisée en ce que la substance pharmaceutiquement active est choisie parmi les antihistaminiques, et notamment l'ASTEMIZOLE, le MALEATE de CHLORFENAMINE, l'AZATADINE et parmi les antagonistes des récepteurs H₂, notamment la FAMOTIDINE.

10. Microsphère selon la revendication 5 caractérisée en ce que la substance pharmaceutiquement active est choisie parmi les stéroïdes, et notamment la DESAMETHASONE, la BETAMETHASONE, le 17-β-ESTRADIOL.

11. Microsphère selon la revendication 5 caractérisée en ce que la substance pharmaceutiquement active est choisie parmi les analgésiques, et notamment l'INDOMETHACINE et le NAPROXENE.

12. Procédé pour améliorer le controle des propriétés pharmacocinétiques et pharmacologiques d'une substance pharmaceutiquement active injectable, ladite substance pharmaceutiquement active étant susceptible d'être administrée par injection parentérale à un mammifère, consistant à :
- Mettre ladite substance sous forme de microsphères solides non poreuses d'un diamètre compris entre 5 et 300 micrometers (microns) contenant ladite substance pharmaceutiquement active dans une structure sphérique formée par au moins une substance-support pharmacologiquement inactive, ladite substance-support formant la structure sphérique étant naturellement présente dans l'organisme dudit mammifère, étant stable à l'état solide jusqu'à une température d'au moins 60°C et dans le milieu physiologique receveur dudit mammifère, la cinétique de dissolution de la substance-support dans l'organisme du mammifère receveur étant plus lente que la cinétique de libération de la substance active dans ce même organisme.
- Séparer lesdites microsphères en fractions calibrées selon leurs diamètres.

13. Procédé selon la revendication 12, caractérisé en ce que la séparation en fractions est effectuée de manière à ce que plus de 70% desdites microsphères ont des diamètres compris entre 70% et 130% d'un diamètre spécifié.

14. Procédé selon la revendication 13 comprenant les étapes suivantes :
a. dispersion de la substance active dans la substance-support et fusion de la substance-support sous atmosphère inerte.
b. Pulvérisation de la matière fondue en brouillard de gouttelettes sous pression d'atmosphère inerte.
c. Congélation en atmosphère froide.
d. Tri par fraction granulométriques.

15. Procédé selon l'une des revendications 12, 13 ou 14, caractérisé en ce que la substance-support est le cholestérol.

16. Utilisation de microsphères selon l'une quelconque des revendications 1 à 11, calibrées, pour la fabrication d'une formulation destinée à l'administration parentérale par injection.

17. Utilisation selon la revendication 16, caractérisée en ce que le mode d'administration est choisi parmi l'injection hypodermique, l'injection sous-cutanée, l'injection intramusculaire, l'injection intra-articulaire et l'injection intra-rachidienne.

18. Utilisation selon la revendication 16, caractérisée en ce que lesdites microsphères sont présentées sous forme d'une poudre, prête à être mise en suspension au moment de l'emploi dans un vecteur liquide pharmaceutiquement acceptable choisi parmi les solutions aqueuses, notamment une solution saline, et les huiles.

19. Utilisation selon la revendication 16, caractérisée en ce que lesdites microsphères sont présentées sous forme d'une suspension dans un vecteur liquide pharmaceutiquement acceptable dans lequel lesdites substances actives sont sensiblement insolubles.

20. Utilisation de microsphères selon la revendication 1 pour la fabrication d'un anticonceptif destiné à l'injection parentérale mensuelle, caractérisée en ce que ladite formulation comprend des microsphères calibrées de 17-β-estradiol contenues dans une structure de cholestérol.

## Claims

1. A solid, nonporous microsphere of a diameter of between 5 and 300 »m, comprising at least one pharmaceutically active substance contained in a spherical structure formed by at least one pharmacologically inactive carrier substance, said pharmaceutically active substance being capable of being administered by parenteral injection to a mammal, characterized in that said carrier substance forming the spherical structure is naturally present in the receiving organism and is stable in the solid state up to a temperature of at least 60°C and in the physiological receiving medium, and that the kinetics of dissolution of the carrier substance in the receiving mammalian organism is slower than the kinetics of release of the active substance in this same organism.

2. The microsphere as claimed in claim 1, wherein the diameter of said microsphere is between 10 and 100 »m.

3. The microsphere as claimed in claim 2, wherein its composition furthermore comprises pharmaceutically acceptable additives.

4. The microsphere as claimed in one of claims 1 to 3, wherein said carrier substance is selected from in the melted state chemically stable substances, the pharmaceutically active substance is selected from the substances which are chemically stable in the melted carrier substance and wherein said microsphere is obtained by dispersing said phar- maceutically active substance in said carrier substance in the melted state, followed by spraying of the mixture obtained and rapid freezing of the droplets in a cold gas.

5. The microsphere as claimed in claim 4, wherein the carrier substance is chosen from coprosterol, glycocholic acid, cholesterol and cholesterol esters.

6. The microsphere as claimed in claim 5, wherein the pharmaceutically active substance is chosen from among substances which act on the central nervous system such as tranquilizers, in particular LORAZEPAM, HALOPERIDOL and DIAZEPAM, such as antiparkinsonians, in particular BIPERIDEN, TRIHEXYLPHENIDYL HCL, such as anticonvulsants, in particular CLONAZEPAM and such as narcotics, in particular MORPHINE BASE and morphine derivatives.

7. The microsphere as claimed in claim 5, wherein the pharmaceutically active substance is chosen from among substances which act on the neurovegetative system, such as anti-emetics, in particular METHOCLOPRAMIDE, ACEPROMAZINE MALEATE and such as gastrokinetic drugs, in particular DOMPERIDONA.

8. The microsphere as claimed in claim 5, wherein the pharmaceutically active substance is chosen from among peripheral vasodilators, in particular VINCAMINE, NYLIDRIN, FLUNARIZINE, PIZOTIFEN, DIHYDROERGOTAMINE, and bronchitic drugs, in particular BROMOHYDRATE, FENOTEROL, TOLBUTEROL, CLENBUTEROL and SALBUTAMOL.

9. The microsphere as claimed in claim 5, wherein the pharmaceutically active substance is chosen from among antihistaminics and in particular ASTEMIZOLE, CHLORFENAMINE MALEATE, AZATADINE and chosen from among H₂ receptor antagonists, in particular FAMOTIDINE.

10. The microsphere as claimed in claim 5, wherein the pharmaceutically active substance is chosen from among steroids and in particular DEXAMETHASONE, BETAMETHASONE and 17-BETA-ESTRADIOL.

11. The microsphere as claimed in claim 5, wherein the pharmaceutically active substance is chosen from among analgesics and in particular INDOMETHACINE and NAPROXENE.

12. A process for improving the control of the pharmacokinetic and pharmacological properties of an injectable pharmaceutically active substance, said pharmaceutically active substance being capable of being administered by parenteral injection to a mammal, consisting in:
- preparing said substance in the form of solid, nonporous microspheres of a diameter of between 5 and 300 microns, comprising the said pharmaceutically active substance contained in a spherical structure formed by at least one pharmacologically inactive carrier substance, said carrier substance forming the spherical structure being naturally present in said mammalian organism and being stable in the solid state up to a temperature of at least 60°C and in the physiological receiving medium of said mammal, the kinetics of dissolution of the carrier substance in the receiving mammalian organism being slower than the kinetics of release of the active sub- stance in this same organism,
- separating said microspheres into calibrated fractions according to their diameters.

13. The process as claimed in claim 12, wherein the separation into fractions is performed until more than 70% of said microspheres have diameters between 70% and 130% of a specified diameter.

14. The process as claimed in claim 13, comprising the following stages:
a. Dispersing of the active substance in the carrier substance and melting of the carrier substance under inert atmosphere.
b. Spraying of the molten material as a cloud of droplets under pressure in an inert atmosphere.
c. Freezing in a cold inert atmosphere.
d. Separation according to particle size fractions.

15. The process as claimed in anyone of claims 12, 13, or 14, wherein the said carrier substance is cholesterol.

16. The use of microspheres as claimed in anyone of claims 1 to 11, in calibrated form, for the manufacture of a formulation intended for parenteral administration by injection.

17. The use as claimed in claim 15, wherein the mode of administration is chosen from among hypodermic injection, subcutaneous injection, intramuscular injection, intra-articular injection and intra-rachidian injection.

18. The use as claimed in claim 16, wherein said microspheres are provided in the form of a powder ready for preparing into a suspension at the time of use in a pharmaceutically acceptable liquid vector chosen from among aqueous solutions, in particular a saline solution, and oils.

19. The use as claimed in claim 16, wherein said microspheres are provided in the form of a suspension in a pharmaceutically acceptable liquid vector in which said active substance is substantially insoluble

20. The use of microspheres as claimed in claim 1 for the manufacture of a contraceptive intended for monthly parenteral injection, wherein said formulation comprises calibrated 17-β-estradiol microspheres contained in a cholesterol structure.

## Patentansprüche

1. Feste, nicht poröse Mikrokugel, mit einem Durchmesser zwischen 5»m und 300»m, die aus wenigstens einer pharmazeutisch aktiven Substanz, welche in einer, durch eine pharmakologisch inaktive Trägersubstanz gebildeten sphärischen Struktur enthalten ist, besteht, wobei die genannte pharmazeutisch aktive Substanz einem Säugetier durch parenterale Injektion verabreicht werden kann, dadurch gekennzeichnet, dass die genannte Trägersubstanz, die die sphärische Struktur bildet, natürlich in dem empfangenden Organismus vorhanden ist, im festen Zustand bis zu einer Temperatur von wenigstens 60°C und in dem empfangenden physiologischen Milieu stabil ist, und dass die Auflösungskinetik der Trägersubstanz im Organismus des empfangenden Säugetiers langsamer ist als die Kinetik des Freiwerdens der aktiven Substanz in demselben Organismus.

2. Mikrokugel gemäss Anspruch 1, dadurch gekennzeichnet, dass der Durchmesser der genannten Mikrokugel zwischen 10 »m und 100 »m beträgt.

3. Mikrokugel gemäss Anspruch 2, dadurch gekennzeichnet, dass ihre Zusammensetzung ebenfals pharmazeutisch annehmbare Zusatzsubstanzen einbegreift.

4. Mikrokugel gemäss einem der Ansprüche 1 bis 3, dadurch gekennzeichnet, dass die genannte Trägersubstanz zwischen den Substanzen ausgewählt wird, die chemisch stabil in geschmolzenem Zustand sind, dass die pharmazeutisch aktive Substanz zwischen den Substanzen, die chemisch stabil in der geschmolzenen Trägersubstanz sind, ausgewählt wird, und dass die genannte Mikrokugel durch Dispersion der genannten pharmazeutisch aktiven Substanz in der genannten geschmolzenen Trägersubstanz, Zerstäubung der erhaltenen Mischung und schnelles Einfrieren der Tröpfchen in einem kalten Gas erhalten wird.

5. Mikrokugel gemäss Anspruch 4, dadurch gekennzeichnet, dass die Trägersubstanz zwischen Koprosterin, Glycocholsäure, Cholesterin und Cholesterinestern ausgewählt wird.

6. Mikrokugel gemäss Anspruch 5, dadurch gekennzeichnet, dass die pharmazeutisch aktive Substanz zwischen den auf das zentrale Nervensystem wirkenden Substanzen, als Beruhigungssubstanzen, insbesondere Lorazepam, Haloperidol und Diazepam, als anti-Parkinson wirkende Substanzen, insbesondere Biperiden, Trihexylphenidyl HCL, als anti-Krampfmittel, insbesondere Clonazepam, als Narkotika, insbesondere Basismorphium und Morphiumderivate ausgewählt wird.

7. Mikrokugel gemäss Anspruch 5, dadurch gekennzeichnet, dass die pharmazeutisch aktive Substanz zwischen den auf das vegetative Nervensystem als anti-Brechmittel wirkende, insbesondere Methoclopramid, Azepromazin-Maleat, oder als gastrokinetisch wirkenden Substanzen, insbesondere Domperidona, ausgewählt wird.

8. Mikrokugel gemäss Anspruch 5, dadurch gekennzeichnet, dass die pharmazeutisch aktive Substanz zwischen den die peripheren Gefässe erweiternden Substanzen, insbesondere Vincamin, Nylidrin, Funarizin, Pizotifen, Dihydroergotamin und den bronchialerweiternden Substanzen, insbesondere Bromohydrat, Fenoterol, Tolbuterol, Clenbuterol und Solbutamol ausgewählt wird.

9. Mikrokugel gemäss Anspruch 5, dadurch gekennzeichnet, dass die pharmazeutisch aktive Substanz unter den anti-Histaminika, insbesondere Astemizol, Chlorfenamin-Maleat, Azatadin und den H₂-Rezeptoren-Antagonisten, insbesondere Famotidin, ausgewählt wird.

10. Mikrokugel gemäss Anspruch 5, dadurch gekennzeichnet, dass die pharmazeutisch aktive Substanz zwischen den Steroïden, insbesondere Desamethason, Betamethason und 17-Beta-Estradiol ausgewählt wird.

11. Mikrokugel gemäss Anspruch 5, dadurch gekennzeichnet, dass die pharmazeutisch aktive Substanz zwischen den schmerzstillenden Substanzen, insbesondere Indomethacin und Naproxen ausgewählt wird.

12. Verfahren, um die Kontrolle über die pharmakinetischen und pharmakologischen Eigenschaften einer injizierbaren pharmazeutisch aktiven Substanz zu verbessern, wobei die genannte pharmazeutisch aktive Substanz einem Säugetier durch parenterale Injektion verabreicht werden kann, dass darin besteht, dass man die genannte Substanz in Form von festen, nicht porösen Mikrokugeln mit einem Durchmesser zwischen 5 »m und 300 »m versetzt, die die genannte aktive pharmazeutische Substanz in einer sphärischen Struktur enthalten, welche aus wenigstens einer pharmakologisch inaktiven Trägersubstanz gebildet ist, wobei die genannte, die sphärische Struktur bildende Trägersubstanz, natürlich im Organismus des genannten Säugetiers vorhanden ist, im festen Zustand bis zu einer Temperatur von wenigstens 60°C und im empfangenden, physiologischen Medium des genannten Säugetiers stabil ist, wobei die Auflösungskinetik der Trägersubstanz im Organismus des empfangenden Säugetiers langsamer ist als die Freisetzungskinetik der aktiven Substanz in demselben Organismus, und dass man die genannten Mikrokugeln, ihrem Durchmesser nach, in kalibrierte Fraktionen auftrennt.

13. Verfahren gemäss Anspruch 12, dadurch gekennzeichnet, dass die Auftrennung in Fraktionen so erfolgt, dass mehr als 70% der genannten Mikrokugeln Durchmesser aufweisen, die zwischen 70% und 130% eines vorgeschriebenen Durchmessers betragen.

14. Verfahren gemäss Anspruch 13, mit folgenden Schritten: a) Dispersion der aktiven Substanz in der Trägersubstanz und Schmelzen der Trägersubstanz unter inerter Atmosphäre,
b) Zerstäuben der geschmolzenen Materie in einen Tröpfchennebel, unter Druck, in inerter Atmosphäre,
c) Einfrieren in kalter Atmosphäre,
d) Auftrennnung in korngrössenmässige Fraktionen.

15. Verfahren gemäss einem der Ansprüche 12, 13 oder 14, dadurch gekennzeichnet, dass die Trägersubstanz Cholesterin ist.

16. Verwendung von kalibrierten Mikrokugeln gemäss irgend einem der Ansprüche 1 bis 11, zur Herstellung eines Arzneimittels, welches zur parenteralen Verabreichung durch Injizieren bestimmt ist.

17. Verwendung gemäss Anspruch 15, dadurch gekennzeichnet, dass die Verabreichungsart zwischen der Unterhaut, der Subkutanen-, der Intramuskularen-, der Intraartikularen- und der Intravirbelkanalinjektion ausgewählt ist.

18. Verwendung gemäss Anspruch 16, dadurch gekennzeichnet, dass die genannten Mikrokugeln in Form eines Pulvers aufbereitet werden, das fertig zum Suspendieren, zur Gebrauchszeit, in einem pharmazeutisch annehmbaren flüssigen Träger ist, der zwischen den wässrigen Lösungen, insbesondere den Salzlösungen, und den Oelen ausgewählt wird.

19. Verwendung gemäss Anspruch 16, dadurch gekennzeichnet, dass die genannten Mikrokugeln in Form einer Suspension, in einem pharmazeutisch annehmbaren flüssigen Träger, in welchem die genannten Mikrokugeln wesentlich unlösbar sind, dargeboten werden.

20. Verwendung von Mikrokugeln gemäss Anspruch 1, zur Herstellung eines empfängnisverhütenden Mittels, das zur monatlichen Parenteral-Injektion bestimmt ist, dadurch gekennzeichnet, dass das genannte Mittel kalibrierte Kugeln aus 17-Beta-Estradiol, enthalten in einer Cholesterin-Struktur, einbegreift.
